# EUROPEAN PATENT SPECIFICATION

(11) **EP 1 881 984 B1**
(45) Date of publication and mention of the grant of the patent: **04.04.2012**
(21) Application number: 06747700.0
(22) Date of filing: 22.05.2006
(51) Int. Cl.: C07D 473/00, A61K 31/52, A61K 31/522, A61P 31/00, C07D 473/04, C07D 473/18, C07D 473/30, C07D 473/34

(54) **INHIBITORS OF NUCLEOSIDE PHOSPHORYLASES AND NUCLEOSIDASES**
INHIBITOREN VON NUKLEOSIDPHOSPHORYLASEN UND -NUKLEOSIDASEN
INHIBITEURS DE NUCLEOSIDE PHOSPHORYLASES ET DE NUCLEOSIDASES

(30) Priority: 20.05.2005 NZ 54016005
(43) Date of publication of application: 30.01.2008
(73) Proprietor: ALBERT EINSTEIN COLLEGE OF MEDICINE OF YESHIVA UNIVERSITY, a division of YESHIVA UNIVERSITY, Bronx New York 10461 (US); INDUSTRIAL RESEARCH LIMITED, Lower Hutt 6009 (NZ)
(72) Inventor: EVANS, Gary Brian, Normandale Lower Hutt (NZ); FURNEAUX, Richard Hubert, Wil ton Wellington (NZ); SCHRAMM, Vern L., New Rochelle, NY 10805 (US); TYLER, Peter Charles, Northla nd Wellington (NZ); MEE, Simon Peter Harold, Peton e Lower Hutt (NZ); ZUBKOVA, Olga Vladimirovna, Karo ri Wellington (NZ)
(74) Representative: Mathys & Squire LLP
(86) International application number: PCT/NZ2006/000123
(87) International publication number: WO 2006/123953

(56) References cited:
- WO-A1-02/18371
- WO-A1-03/080620
- WO-A1-2004/018496
- WO-A2-00/61783
- LEWANDOWICZ A. ET AL.: 'Energetic Mapping of Transition State Analogue Interactions with Human and Plasmodium falciparum Purine Nucleotide Phosphorylases' JOURNAL OF BIOLOGICAL CHEMISTRY vol. 280, no. 34, 2005, pages 30320 - 30328, XP003004125

## Description

### TECHNICAL FIELD

This invention relates generally to certain nucleoside analogues, the use of these compounds as pharmaceutical, pharmaceutical compositions containing the compounds, processes for preparing the compounds, and the use of the compounds in treating diseases or conditions in which it is desirable to inhibit purine phosphoribosyltransferase, purine nucleoside phosphorylase, 5'-methylthioadenosine phosphorylase, 5'-methytthioadenosine nucleosidase and/or nucleoside hydrolase.

### BACKGROUND

US 5,985,848, US 6,086,722 and US 6,228,847 describe nucleoside analogues that are inhibitors of purine nucleoside phosphorylase (PNP) and purine phosphoribosyltransferases (PPRT). The analogues are useful in treating parasitic infections, T-cell malignancies, autoimmune diseases and inflammatory disorders. The analogues are also useful for immunosupression in organ transplantation.

WO 2000/061783 describes a process for preparing certain PNP inhibitor compounds. This application recognizes the compounds as PNP inhibitors and addresses a need for simpler method of preparing them. WO 2002/018371 discloses further nucleoside analogues that are inhibitors of PNP and PPRT.

Certain nucleoside analogues have also been identified as potent inhibitors of 5'-methylthioadenosine phosphorylase (MTAP) and 5'-methylthioadenosine nucleosidase (MTAN). These are the subject of WO 2003/080620 and WO 2004/018496. (MTAN). These are the subject of WO 2003/080620 and WO 2004/018496.

PNP catalyses the phosphorolytic cleavage of ribo- and deoxyribonucleosides, for example those of guanine and hypoxanthine, to give the corresponding sugar-1-phosphate and guanine, hypoxanthine or other purine bases.

Humans deficient in purine nucleoside phosphorylase (PNP) suffer a specific T-cell immunodeficiency due to an accumulation of dGTP which prevents proliferation of stimulated T lymphocytes. Inhibitors against PUP are therefore immunosuppressive, and are active against T-cell malignancies and T-cell proliferative disorders.

Nucleoside hydrolases (NH) catalyse the hydrolysis of nucleosides. These enzymes are not found in mammals but are required for nucleoside salvage in some protozoan parasites. Some protozoan parasites use nucleoside phosphorylases either instead of or in addition to nucleoside hydrolases for this purpose. Inhibitors of nucleoside hydrolases and phosphorylases can be expected to interfere with the metabolism of the parasite and can therefore be usefully employed against protozoan parasites.

MTAP and MTAN function in the polyamine biosynthesis pathway, in purine salvage in mammals, and in the quorum sensing pathways in bacteria. MTAP catalyses the reversible phosphorolysis of 5'-methylthioadenosine (MTA) to adenine and 5-methylthio-α-D-ribose-1-phosphate (MTR-1P). MTAN catalyses the reversible hydrolysis of MTA to adenine and 5-methylthio-α-D-ribose and of S-adenosyl-L-homocysteine (SAH) to adenine and S-ribosyl-homocysteine (SRH). The adenine formed is subsequently recycled and converted into nucleotides. Essentially, the only source of free adenine in the human cell is a result of the action of these enzymes. The MTR-1P is subsequently converted into methionine by successive enzymatic actions.

MTA is a by-product of the reaction involving the transfer of an aminopropyl group from decarboxylated S-adenosylmethionine to putrescine during the formation of spermidine. The reaction is catalyzed by spermidine synthase. The spermidine synthase is very sensitive to product inhibition by accumulation of MTA. Therefore, inhibition of MTAP or MTAN severely limits the polyamine biosynthesis and the salvage pathway for adenine in the cells. Likewise, MTA is the by-product of the bacterial synthesis of acylated homoserine lactones from S-adenosylmethionine (SAM) and acyl-acyl carrier proteins in which the subsequent lactonization causes release of MTA and the acylated homoserine lactone. The acylated homoserine lactone is a bacterial quorum sensing molecule in bacteria that is involved in bacterial virulence against human tissues. Recent work has identified a second communication system (autoinducer 2, Al-2) that is common to both Gram-positive and Gram-negative bacteria and thus has been proposed as a "universal signal" which functions in interspecies cell-to-cell communication. Again, MTAN generates S-ribosyl-homocysteine (SRH) that is the precursor of Al-2. Inhibition of MTAN or MTAP in microbes will prevent MTA removal and subject the pathway to product inhibition, thereby decreasing production of the quorum sensing pathway and decreasing the virulence of microbial infections. Inhibition of MTAN in microbes will prevent the formation of SRH, decreasing the production of the second quorum sensing pathway.

MTAP deficiency due to a genetic deletion has been reported with many malignancies. The loss of MTAP enzyme function in these cells is known to be due to homozygous deletions on chromosome 9 of the closely linked MTAP and *p16*/*MTS1* tumour suppressor gene. As absence of *p16*/*MTS1* is probably responsible for the tumour, the lack of MTAP activity is a consequence of the genetic deletion and is not causative for the cancer. However, the absence of MTAP alters the purine metabolism in these cells so that they are mainly dependent on the *de novo* pathway for their supply of purines. That makes these cells unusually sensitive to inhibitors like methotrexate, alanosine and azaserine, that block the *de novo* pathway. Therefore, a combination therapy of methotrexate, alanosine or azaserine with an MTAP inhibitor will have unusually effective anti-tumour properties.

MTAP inhibitors would also be very effective against parasitic infection such as malaria that infects red blood cells (RBCs), as they lack the *de novo* pathway for purine biosynthesis. Protozoan parasites depend entirely upon the purines produced by the salvage pathway for their growth and propagation. MAP inhibitors will therefore kill these parasites without having any negative effect on the host RBCs, as RBCs are terminally differentiated cells and they do not synthesize purines, produce polyamines or multiply.

The imino sugar part of the compounds described in the patent specifications referred to above has the nitrogen atom located between C-1 and C-4 so as to form 1,4-dideoxy-1,4-imino-D-ribitol compounds. The location of the nitrogen atom in the ribitol ring may be critical for binding to enzymes. In addition, the location of the link between the sugar moiety and the nucleoside base analogue may be critical for enzyme inhibitory activity. The compounds described above have that link at C-1 of the sugar ring.

The applicants have also developed other nucleoside phosphorylase and nucleosidase inhibitors, where the location of the nitrogen atom in the sugar ring is varied and, additionally, where two nitrogen atoms form part of the sugar ring. Alternative modes of linking the sugar part and the base analogue have also been investigated, resulting in a class of inhibitors where the sugar moiety is linked to the nucleoside base analogue via a methylene bridge. These other inhibitors are described in WO 2004/018496.

However, there remains an ongoing need for new inhibitors of PNP, PPRT, MTAP, MTAN, and NH. In particular, the applicants have now found that ethylene-linked analogues of the abovementioned methylene-linked compounds are surprisingly potent inhibitors of PNP. The same class of compounds are anticipated to be inhibitors of PPRT, MTAP, MTAN, and NH.

It is therefore an object of the present invention to provide compounds that are inhibitors of PNP, PPRT, MTAP, MTAN, and/or NH, or to at least provide a useful choice.

### STATEMENTS OF INVENTION

Accordingly, in a first aspect, the present invention provides a compound of the formula (I): 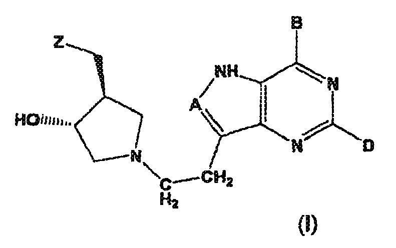 where:
A is N or CH;
B is OH or NH₂;
D is H, OH, NH₂ or SCHg; and
Z is OH or SQ, where Q is an alkyl, aralkyl, or aryl group, each of which is optionally susbtituted with one or more substituents selected from an alkyl group, an alkoxy group (wherein the alkyl group is as defined above), a halogen atom, an amino group, a carboxylic acid group, an carboxylate alkyl ester group, and an alkylthio group;
or a tautomer thereof; or a pharmaceutically acceptable salt thereof; or an ester form thereof.

Preferably A is CH. Alternatively, A may be N.

It is also preferred that B is OH. Alternatively, B is NH₂.

It is further preferred that D is H. Alternatively, D may preferably be NH₂, OH, or SCH₃.

In some preferred compounds of the invention Z is OH. in other preferred compounds Z is SQ.

Further preferred compounds of the invention are those where Z is OH, A is CH, B is OH, and D is H or NH₂.

Other preferred compounds of the invention are those where Z is SQ, A is CH, B is NH₂, and D is H.

Preferred compounds of the invention include:
(i) (3S,4S)-1-[(9-deazaadenin-9-yl)ethyl]-3-hydroxy-4-(hydroxymethyl)-pyrrolidine;
(ii) (3S,4R)-1-[(9-deazaadenin-9-yl)ethyl]-3-hydroxy-4-(methylthiomethyl)-pyrrolidine;
(iii) (3S,4R)-1-[(9-deazaadenin-9-yl)ethyl]-3-hydroxy-4-(ethylthiomethyl)-pyrrolidine;
(iv) (3S,4R)-1-[(9-deazaadenin-9-yl)ethyl]-3-hydroxy-4-(2-fluoroethylthiomethyl)-pyrrolidine;
(v) (3S,4R)-1-[(9-deazaadenin-9-yl)ethyl]-3-hydroxy-4-(2-hydroxyethylthiomethyl)-pyrrolidine;
(vi) (3S,4R)-1-[(9-deazaadenin-9-yl)ethyl]-3-hydroxy-4-(propylthiomethyl)-pyrrolidine;
(vii) (3S,4R)-1-[(9-deazaadenin-9-yl)ethyl]-3-hydroxy-4-(isopropylthiomethyl)-pyrrolidine;
(viii) (3S,4R)-1-[(9-deazaadenin-9-yl)ethyl]-3-hydroxy-4-(butylthiomethyl)-pyrrolidine;
(ix) (3S,4R)-1-[(9-deazaadenin-9-yl)ethyl]-3-hydroxy-4-(cyclohexylylthiomethyl)-pyrrolidine;
(x) (3S,4R)-1-[(9-deazaadenin-9-yl)ethyl]-3-hydroxy-4-(cyclohexyimethylthiomethyl)-pyrrolidine;
(xi) (3S,4R)-1-[(9-deazaadenin-9-yl)ethyl]-3-hydroxy-4-(cyclopentylthiomethyl)-pyrrolidine;
(xii) (35,4R)-1-[(9-deazaadenin-9-yl)ethyl]-3-hydroxy-4-(phenylthiomethyl)-pyrrolidine;
(xiii) (3S,4R)-1-[(9-deazaadenin-9-yl)ethyl]-3-hydroxy-4-(4-fluorophenylthiomethyl)-pyrrolidine;
(xiv) (3S,4R)-1-[(9-deazaadenin-9-yl)ethyl]-3-hydroxy-4-(4-chlorophenylthiomethyl)-pyrrolidine;
(xv) (3S,4R)-1-[(9-deazaadenin-9-yl)ethyl]-3-hydroxy-4-(3-chlorophenylthiomethyl)-pyrrolidine;
(xvi) (3S,4R)-1-[(9-deazaadenin-9-yl)ethyl]-3-hydroxy-4-(4-methylphenylthiomethyl)-pyrrolidine;
(xvii) (3S,4R)-1-[(9-deazaadenin-9-yl)ethyl]-3-hydroxy-4-(3-methylphenylthiomethyl)-pyrrolidine;
(xviii) (3S,4R)-1-[(9-deazaadenin-9-yl)ethyl]-3-hydroxy-4-(benzylthiomethyl)-pyrrolidine;
(xix) (3S,4S)-1-[(9-deazaguanin-9-yl)ethyl]-3-hydroxy-4-(hydroxymethyl)-pyrrolidine;
(xx) (3S,4R)-1-[(9-deazaguanin-9-yl)ethyl]-3-hydroxy-4-(methylthiomethyl)-pyrrolidine;
(xxi) (3S,4S)-1-[(9-deazahypoxanthin-9-yl)ethyl]-3-hydroxy-4-(hydroxymethyl)-pyrrolidine;
(xxii) (3S,4R)-1-[(9-deazahypoxanthin-9-yl)ethyl]-3-hydroxy-4-(methylthiomethyl)-pyrrolidine;
(xxiii) (3S,4S)-1-[(9-deazaxanthin-9-yl)ethyl]-3-hydroxy-4-(hydroxymethyl)-pyrrolidine;
(xxiv) (3S,4S)-1-[(9-deazaxanthin-9-yl)ethyl]-3-hydroxy-4-(methylthiomethyl)-pyrrolidine;
(xxv) (3S,4S)-1-[(8-aza-9-deazaadenin-9-yl)ethyl]-3-hydroxy-4-(hydroxymethyl)-pyrrolidine;
(xxvi) (35,45)-1-[(8-aza-9-deazaadenin-9-yl)ethyl]-3-hydroxy-4-methyl-pyrrolidine;
(xxvii) (3S,4R)-1-[(8-aza-9-deazaadenin-9-yl)ethyl]-3-hydroxy-4-(benzylthiomethyl)-pyrrolidine;
(xxviii) (3S,4R)-1-[(8-aza-9-deazaadenin-9-yl)ethyl]-3-hydroxy-4-(methylthiomethyl)-pyrrolidine;
(xxix) (3S,4R)-1-[(8-aza-9-deazaadenin-9-yl)ethyl]-3-hydroxy-4-(ethylthiomethyl)-pyrrolidine;
(xxx) (3S,4R)-1-[(8-aza-9-deazaadenin-9-yl)ethyl]-3-hydroxy-4-(propylthiomethyl)-pyrrolidine;
(xxxi) (3S,4R)-1-[(8-aza-9-deazaadenin-9-yl)ethyl]-3-hydroxy-4-(isopropylthiomethyl)-pyrrolidine;
(xxxii) (3S,4R)-1-[(8-aza-9-deazaadenin-9-yl)ethyl]-3-hydroxy-4-(butylthiomethyl)-pyrrolidine;
(xxxiii) (3S,4R)-1-[(8-aza-9-deazaadenin-9-yl)ethyl]-3-hydroxy-4-(phenylthiomethyl)-pyrrolidine;
(xxxiv) (3S,4R)-1-[(8-aza-9-deazaadenin-9-yl)ethyl]-3-hydroxy-4-(4-fluorophenylthiomethyl)-pyrrolidine;
(xxxv) (3S,4R)-1-[(8-aza-9-deazaadenin-9-yl)ethyl)-3-hydroxy-4-(4-chlorophenylthiomethyl)-pyrrolidine;
(xxxvi) (3S,4R)-9-[(8-aza-9-deazaadenin-9-yl)ethyl]-3-hydroxy-4-(3-chlorophenylhiomethyl)-pyrrolidine;
(xxxvii) (3S,4R)-1-[(8-aza-9-deazaadenin-9-yl)ethyl]-3-hydroxy-4-(4-methylphenylthiomethyl)-pyrrolidine;
(xxxviii) (3S,4R)-1-[(8-aza-9-deazaadenin-9-yl)ethyl]-3-hydroxy-4-(3-methylphenylthiomethyl)-pyrrolidine;
(xxxix) (38,4S)-1-(8-aza-9-deazaguanin-9-yl)ethyl]-3-hydroxy-4-(hydroxymethyl)-pyrrolidine;
(xl) (3S,4S)-1-[(8-aza-9-deazaguanin-9-yl)ethyl]-3-hydroxy-4-methyl-pyrrolidine;
(xli) (35,4S)-1-[(8-aza-9-deazaguanin-9-yl)ethyl]-3-hydroxy-4-(methylthiomethyl)-pyrrolidine;
(xlii) (3S,4S)-1-[(8-aza-9-deazahypoxanthin-9-yl)ethyl]-3-hydroxy-4-(hydroxymethyl)-pyrrolidine;
(xliii) (3S,4S)-1-[(8-aza-9-deazahypoxanthin-9-yl)ethyl]-3-hydroxy-4-methyl-pyrrolidine;
(xliv) (3S,4S)-1-[(8-aza-9-deazahypoxanthin-9-yl)ethyl]-3-hydroxy-4-(methylthiomethyl)-pyrrolidine;
(xlv) (3S,4S)-1-[(8-aza-9-deazaxanthin-9-yl)ethyl]-3-hydroxy-4-(hydroxymethyl)-pyrrolidine; and
(xlvi) (3S,4S)-1-[(8-aza-9-deazaxanthin-9-yl)ethyl]-3-hydroxy-4-(methylthiomethyl)-pyrrolidine.

In a second aspect of the invention there is provided a pharmaceutical composition comprising a pharmaceutically effective amount of a compound of formula (I).

The compounds may be useful in the treatment of a disease or condition in which it is desirable to inhibit purine phosphoribosyltransferase, purine nucleoside phosphorylase, 5'-methylthioadenosine phosphorylase. 5'-methylthioadenosine nucleosidase and/or nucleoside hydrolase.

The diseases or conditions include cancer, bacterial and protozoal infections, and T-cell mediated diseases such as psoriasis, arthritis and transplant rejection.

In a further aspect of the invention there is provided the use of a compound of formula (I) in the manufacture of a medicament for the treatment of one or more of these diseases or conditions.

In still a further aspect of the invention there is provided a method of preparing a compound of formula (I).

### DETAILED DESCRIPTION

### Definitions

The term "alkyl" is intended to include both straight- and branched-chain alkyl groups. The same terminology applies to the non-aromatic moiety of an aralkyl radical. Examples of alkyl groups include: methyl group, ethyl group, *n*-propyl group, *iso*-propyl group, *n*-butyl group, *iso*-butyl group, sec-butyl group, *t*-butyl group, *n*-pentyl group, 1,1-dimethylpropyl group, 1,2-dimethylpropyl group, 2,2-dimethylpropyl group, 1-ethylpropyl group, 2-ethylpropyl group, *n*-hexyl group and 1-methyl-2-ethylpropyl group.

The term "aryl" means an aromatic radical having 4 to 18 carbon atoms and includes heteroaromatic radicals. Example include monocyclic groups, as well as fused groups such as bicyclic groups and tricyclic groups. Some examples include phenyl group, indenyl group, 1-naphthyl group, 2-naphthyl group, azulenyl group, heptalenyl group, biphenyl group, indacenyl group, acenaphthyl group, fluorenyl group, phenalenyl group, phenanthrenyl group, anthracenyl group, cyclopentacyclooctenyl group, benzocyclooctenyl group, pyridyl group, pyrrolyl group, pyridazinyl group, pyrimidinyl group, pyrazinyl group, triazolyl group, tetrazolyl group, benzotriazolyl group, pyrazolyl group, imidazolyl group, benzimidazolyl group, indolyl group, isoindolyl group, indolizinyl group, purinyl group, indazolyl group, furyl group, pyranyl group, benzofuryl group, isobenzofuryl group, thienyl group, thiazolyl group, isothiazolyl group, benzothiazolyl group, oxazolyl group, and isoxazolyl group.

The term "aralkyl" means an alkyl radical bearing an aryl substituent

The term "halogen" includes fluorine, chlorine, bromine and iodine.

Prodrugs of compounds of formula (I) may be prepared by modifying functional groups present in the compounds in such a way that the modifications are cleaved *in vivo* to give the parent compound. The term "prodrug" as used herein means a pharmacologically acceptable derivative of the compound of formula (I), such that an *in vivo* biotransformation of the derivative gives the compound as defined in formula (I).

The term "pharmaceutically acceptable salts" is intended to apply to non-toxic salts derived from inorganic or organic acids, including, for example, the following acid salts: acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, formate, fumarate, glucoheptanoate, glycerophosphate, glycolate, hemisulfate, heptanoate, hexanoate, hydrochloride, hydrobromide, hydroiodide, 2-hydroxyethanesulfonate, lactate, maleate, malonate, methanesulfonate, 2-naphthalenesulfonate, nicotinate, nitrate, oxalate, palmoate, pectinate, persulfate, 3-phenylpropionate, phosphate, picrate, pivalate, propionate, p-toluenesulfonate, salicylate, succinate, sulfate, tartrate, thiocyanate, and undecanoate.

The term "patient" includes human and non-human animals.

### Description of Inhibitors Compounds

The ethylene-linked compounds of the invention are surprisingly potent inhibitors of PNP. A class of PNP inhibitor compounds containing methylene linkages is described in the applicants' PCT application WO 2004/018496**.** The methylene-linked compounds were designed to match the fully dissociated transition states of human PNP and *Plasmodium falciparum* PNP. The applicants have carried out detailed investigations of this methylene-linked class.

Based on their particular knowledge of the PNP enzyme, and the activities of the methylene-linked compounds, the applicants would not have predicted that ethylene-linked compounds would be potent PNP inhibitors, or would even exhibit PNP inhibitory activity at all. It was previously considered that the presence of the extra carbon atom in the linkage would have rendered the ethylene class inactive. It was thought that the inclusion of an extra carbon atom in the linkage would elongate the distance between the ribose mimic (the amine moiety) and the base moiety beyond the length that had been found to be optimum for inhibition of the PNP enzyme. The prior art and the applicants' previous special knowledge of the PNP enzyme actually taught away from synthesising and investigating the activities of the ethylene-linked compounds. However, despite the linkage being outside of the predicted optimal length, the compounds of the invention prove to be surprisingly potent inhibitors of human PNP. Indeed, one compound of the invention (Compound 1) has a Kᵢ* for human PNP of 0.46 ± 0.05 nM, a potency sufficient to have therapeutic potential.

### Synthesis of Inhibitor Compounds

The compounds may be prepared by any method. However, preferably they are prepared by independently synthesising the amine moiety and the base part, and then linking the base part to the nitrogen atom in the ring of the amine moiety. In one preferred embodiment, the ethylene linkage is constructed on the base part in the form of a 2-substituted acetaldehyde moiety, and then linked to the amine moiety by way of a reductive amination reaction.

### General Aspects

The compounds of the invention are useful in both free base form and in the form of salts.

It will be appreciated that the representation of a compound of formula (I), where B and/or D is a hydroxy group, is of the enol-type tautomeric form of a corresponding amide, and this will largely exist in the amide form. The use of the enol-type tautomeric representation is simply to allow fewer structural formulae to represent the compounds of the invention.

The active compounds may be administered to a patient by a variety of routes, including orally, parenterally, by inhalation spray, topically, rectally, nasally, buccally or via an implanted reservoir. The amount of compound to be administered will vary widely according to the nature of the patient and the nature and extent of the disorder to be treated. Typically the dosage for an adult human will be in the range less than 1 to 1000 milligrams, preferably 0.1 to 100 milligrams. The specific dosage required for any particular patient will depend upon a variety of factors, including the patient's age, body weight, general health, sex, etc.

For oral administration the compounds can be formulated into solid or liquid preparations, for example tablets, capsules, powders, solutions, suspensions and dispersions. Such preparations are well known in the art as are other oral dosage regimes not listed here. In the tablet form the compounds may be tableted with conventional tablet bases such as lactose, sucrose and corn starch, together with a binder, a disintegration agent and a lubricant. The binder may be, for example, corn starch or gelatin, the disintegrating agent may be potato starch or alginic acid, and the lubricant may be magnesium stearate. For oral administration in the form of capsules, diluents such as lactose and dried cornstarch may be employed. Other components such as colourings, sweeteners or flavourings may be added.

When aqueous suspensions are required for oral use, the active ingredient may be combined with carriers such as water and ethanol, and emulsifying agents, suspending agents and/or surfactants may be used. Colourings, sweeteners or flavourings may also be added.

The compounds may also be administered by injection in a physiologically acceptable diluent such as water or saline. The diluent may comprise one or more other ingredients such as ethanol, propylene glycol, an oil or a pharmaceutically acceptable surfactant.

The compounds may also be administered topically. Carriers for topical administration of the compounds of include mineral oil, liquid petrolatum, white petrolatum, propylene glycol, polyoxyethylene, polyoxypropylene compound, emulsifying wax and water. The compounds may be present as ingredients in lotions or creams, for topical administration to skin or mucous membranes. Such creams may contain the active compounds suspended or dissolved in one or more pharmaceutically acceptable carriers. Suitable carriers include mineral oil, sorbitan monostearate, polysorbate 60, cetyl ester wax, cetearyl alcohol, 2-octyldodecanol, benzyl alcohol and water.

The compounds may further be administered by means of sustained release systems. For example, they may be incorporated into a slowly dissolving tablet or capsule.

### EXAMPLES

The following examples further illustrate the invention. It is to be appreciated that the invention is not limited to the examples.

### Example 1: Synthesis of (3S,4S)-1-[2-(9-Deaza-hypoxanthin-9-yl)ethyl]-3-hydroxy-4-hydroxymethylpyrrolidine (1) [DAD-Et-Immucillin-H]

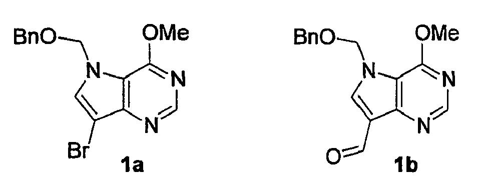

n-Butyllithium (5.30 mL of a 1.3 M solution in hexanes, 6.90 mmol) was added to a solution of bromide **1a** (2.00 g, 5.75 mmol) in diethyl ether (40 mL) and anisole (16 mL) under argon at -78 °C. Thin-layer chromatography confirmed that no starting material remained. Dimethylformamide (4.4 mL, 57.5 mmol) was added and the mixture stirred at -78 °C for 30 minutes then the mixture was allowed to warm to room temperature. Dichloromethane (200 mL) was added and the solution was washed with water (100 mL), dried and the solvent was removed. The residue was chromatographed on silica gel to give compound **1b** (1.20 g, 70%) as a white solid.

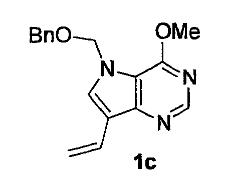

Methyltriphenylphosphonium bromide (1.20 g, 3.37 mmol) was suspended in tetrahydrofuran (25 mL) and cooled to -78 °C under an atmosphere of argon. n-Butyllithium (1.94 mL of a 1.3 M solution in hexanes, 2.52 mmol) was added to give a yellow solution, which was stirred for 15 minutes. Aldehyde **1b** (0.500 g, 1.68 mmol) was added as a solid and the solution was allowed to warm to room temperature then stirred for 2 hours. The solvent was removed and the residue was chromatographed on silica gel to give compound **1c** (0.450 g, 91%) as a pale yellow solid.

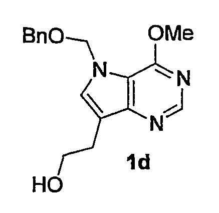

Borane dimethyl sulfide (3.12 mL, 32.9 mmol) was added to a solution of alkene **1c** (0.970 g, 3.29 mmol) in tetrahydrofuran (11 mL) under an atmosphere of argon and the solution was stirred for 18 hours at room temperature. Sodium hydroxide (1.97 g, 49.3 mmol) was dissolved in water (4 mL) then diethyl ether (2 mL) was added slowly to the solution at 0 °C. 30% Aqueous hydrogen peroxide (30% w/w, 8 mL) was added slowly and the mixture was stirred at room temperature for 3 hours. Dichloromethane (100 mL) was added and the mixture was washed with water (100 mL), dried and the solvent was removed. Chromatography of the residue on silica gel gave compound **1d** (0.670 g, 65%) as a white solid.

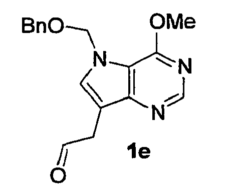

Dess-Martin periodinane (176 mg, 0.415 mmol) was added to a solution of alcohol **1d** (100 mg, 0.319 mmol) in dichloromethane (2 mL) at room temperature giving a yellow precipitate. The mixture was stirred for 10 minutes then chromatographed on silica gel to give compound **1e** (41 mg, 41%). This reaction was repeated with 460 mg of alcohol **1d**, but was left for only 2 minutes with the oxidant and purification was carried out quickly. The yield of compound **1e** increased to 71%, although this material was shown to be not as pure by NMR spectroscopy.

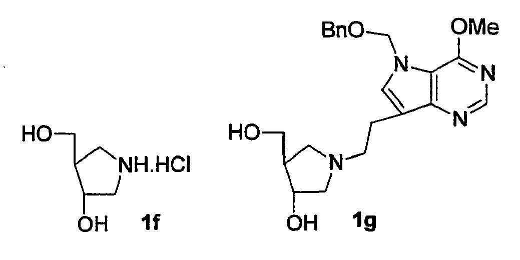

Aldehyde **1e** (110 mg, 0.354 mmol) was added to a solution of amine **1f** (60 mg, 0.389 mmol; reference 1) in methanol (1 mL) at room temperature and the solution stirred for 15 minutes. Sodium cyanoborohydride (29 mg, 0.460 mmol) was then added to the solution, which was stirred for an additional 30 minutes. The mixture was adsorbed onto silica and chromatographed on silica gel giving compound **1g** (20 mg, 14%) as a tan gum.

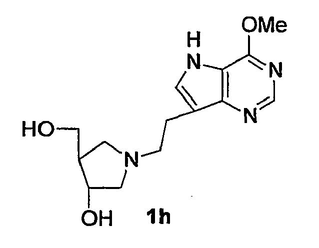

10% Palladium on carbon (20 mg) was added to a solution of **1g** (13 mg, 0.0315 mmol) in ethanol (1 mL) and methanol saturated with ammonia (0.5 mL) and the mixture was stirred under an atmosphere of hydrogen at room temperature for 18 hours. The mixture was filtered and the solvent removed. The residue was chromatographed on silica gel to give compound **1h** (5 mg, 54%) as a tan gum.

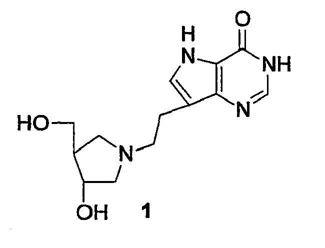

Compound **1h** (4 mg, 0.137 mmol) was heated to reflux in concentrated hydrochloric acid (1 mL) for 2 hours. The solvent was removed to give compound **1** (DAD-Et-Immucillin-H) hydrochloride salt (3 mg, 73%) as a white solid.

### Example 2: Synthesis of (3S,4R)-1-[2-(9-Deaza-adenin-9-yl)ethyl]-3-hydroxy-4-methylthiomethylpyrrolidine (2) [Methylthio-DAD-Et-Immucillin-A]

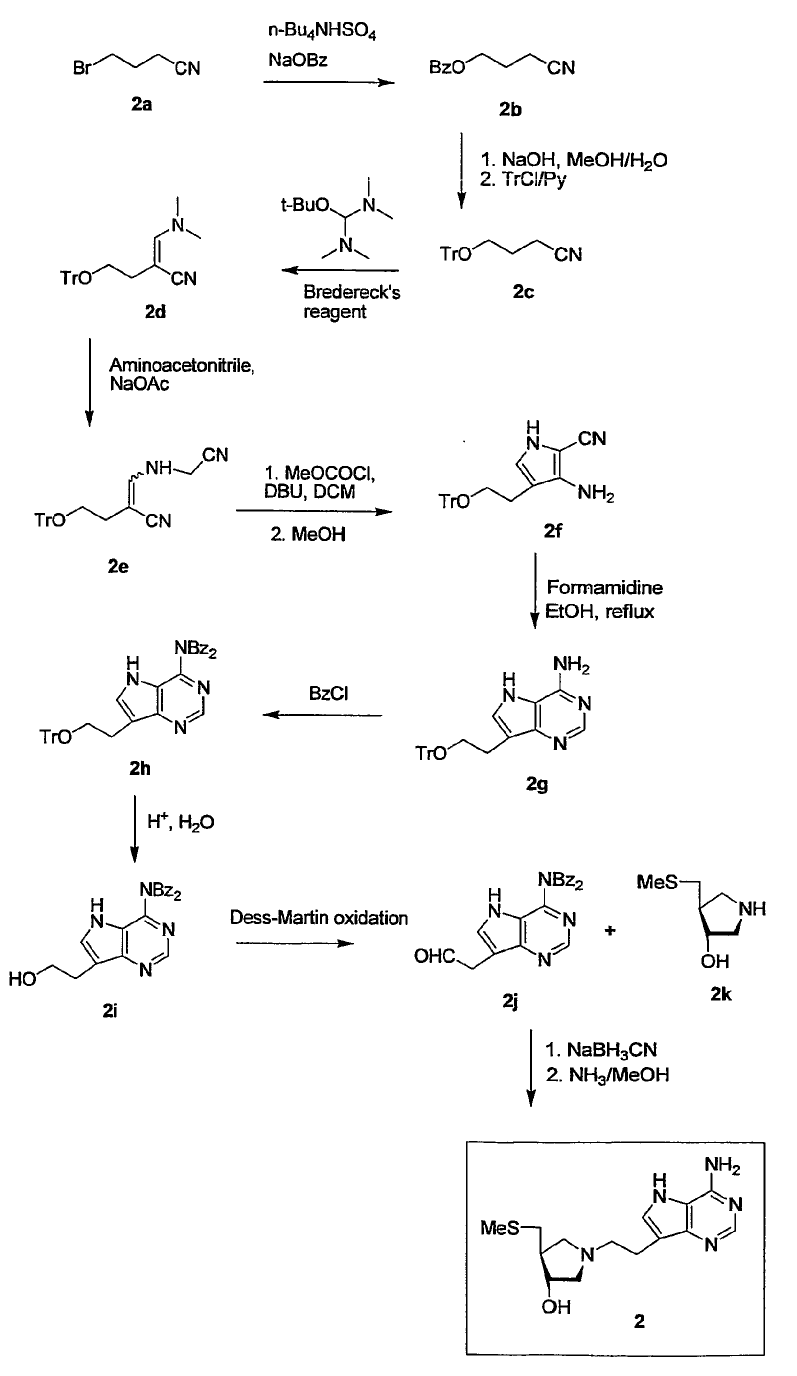

### 3-Cyanopropyl benzoate (2b)

A mixture of bromobutyronitrile (**2a**) (7.45 g, 50.3 mmol), sodium benzoate (14.5 g, 101 mmol), tetrabutylammonium hydrogen sulfate (34.2 g, 101 mmol) and molecular sieves (1 g) in dry acetone (100 ml) was heated under reflux for 4 hrs. The reaction mixture was cooled to RT and filtered through the celite pad and concentrated to dryness. Dichloromethane was added and the mixture was washed with sat. NaHCO₃ followed by water, dried and concentrated. Chromatography (EtOAc : petroleum ether, 1:4) afforded 9.5 g (100%) of (**2a**) as clear syrup. ¹H NMR (CDCl₃) δ 8.02-8.12 (m, 2H), 7.41-7.59 (m, 3H), 4.42 (t, 2H), 2.52 (t, 2H), 2.13 (m, 2H); ¹³C NMR δ 171.5 (C), 166.7 (C), 134.0 (CH), 133.6 (CH), 130.5 (CH), 130.1 (CH), 130.0 (CH), 128.9 (CH), 119.3 (C), 63.1 (CH₂), 25.4 (CH₂), 14.8 (CH₂).

### 4-(Trityloxy)butanenitrile (2c)

To a mixture of benzoate (**2b**) (9.5 g, 50.2 mmol) in methanol (80 ml) was added water (20 ml) and 2M NaOH (10 ml). After stirring for 1 hr at room temperature the reaction mixture was treated with 2M HCl (10 ml), stirred for 15 min and then was concentrated to dryness and dried in *vacuo* to afford a white solid which was used in the next step without further purification. The crude material in dry pyridine was treated with trityl chloride (10.49 g, 37.6 mmol), and the mixture was stirred at room temperature for 17 hrs and concentrated to dryness. Ethyl acetate was added and the mixture was washed twice with water, dried and concentrated. Chromatography (EtOAc : petroleum ether, 1:9) gave trityl derivative (**2c**), 15 g (91%) as a white solid. ¹H NMR (CDCl₃) δ 7.20-7.42 (m, 15H), 3.21 (t, 2H), 2.44 (t, 2H), 1.85-1.9 (m, 2H); ¹³C NMR δ 147.3 (C), 144.3 (C), 128.9 (CH), 128.3 (CH), 127.6 (CH), 127.5 (CH), 119.9 (C), 87.2 (C), 61.7 (CH₂), 26.7 (CH₂), 14.8 (CH₂).

### 2-((Dimethylamino)methylene)-4-(trityloxy)butanenitrile (2d)

Trityl derivative (**2c**) (1 g, 3.05 mmol) was dissolved in dry DMF (15 ml). Bredereck's reagent (0.84 g, 4.84 mmol) was added and the reaction mixture was stirred at 130°C in a flask with a stopper for 1 hr. Bredereck's reagent (0.84 g, 4.84 mmol) was added once more and the mixture was stirred at 130 °C for 2 hrs and concentrated to dryness. Chromatography (EtOAc : petroleum ether, 1:4) gave dimethylamino derivative (**2d**), 0.73 g (62.5 %) as a clear syrup. ¹H NMR (CDCl₃) δ 7.21-7.45 (m, 15H), 6.25 (s, 1H), 3.17 (t, 2H), 3.00 (s, 6H), 2.26 (t, 2H); ¹³C NMR δ 151.2 (CH), 144.7 (C), 129.1 (CH), 128.2 (CH), 127.3 (CH), 122.9 (C), 87.0 (C), 69.7 (C), 63.9 (CH₂), 34.5 (CH₃), 28.4 (CH₂).

### (E/Z)-3-(Cyanomethylamino)-2-(trityloxymethyl)acrylonitrile (2e)

Compound (**2d**) (0.722 g, 1.888 mmol) was dissolved in dry methanol (50 ml). Sodium acetate (1.239 g, 15.10 mmol) and aminoacetonitrile bisulfate (1.164 g, 7.55 mmol) were added and the reaction mixture was stirred under reflux for 5 hrs. The mixture was concentrated to dryness. Chloroform was added, and the reaction mixture was then washed twice with water, dried and concentrated. Chromatography (EtOAc : petroleum ether, 1:2) gave a mixture of cis-trans isomers (**2e**), 0.74 g (100 %) as a pale yellow foam. ¹H NMR (CDCl₃) δ 7.22-7.44 (m, 30H), 6.61 (d, *J* = 12.0 Hz, 1H), 6.43 (d, *J* = 12.6 Hz, 1H), 5.86-5.94 (m, 1H), 4.79-4.85 (m, 1H), 3.89 (d, *J* = 6.1 Hz, 2H), 3.66 (d, *J* = 6.1 Hz, 2H), 3.35 (t, 2H), 3.18 (t, 2H), 2.26-2.32 (m, 4H); ¹³C NMR δ 146.7 (CH), 146.6 (CH), 143.0 (C), 142.2 (C), 127.6 (CH), 127.1 (CH), 126.9 (CH), 126.5 (CH), 126.1 (CH), 121.0 (C), 114.9 (C), 114.8 (C), 87.0 (C), 85.8 (C), 81.3 (C), 79.3 (C), 62.7 (CH₂), 61.5 (CH₂), 34.2 (CH₂), 33.9 (CH₂), 30.1 (CH₂), 27.7 (CH₂).

### 3-Amino-4-(2-(trityloxy)ethyl)-1H-pyrrole-2-carbonitrile (2f)

DBU (1.7 ml, 11.28 mmol) was added to a stirred solution of nitrile (2e) (0.74 g, 1.88 mmol) in dry dichloromethane at room temperature. Methyl chloroformate (0.44 ml, 5.64 mmol) was added drop wise and the reaction mixture was stirred at RT for 17 hrs. Methanol (4 ml) was then added and after 1 hr the resulting solution was diluted with dichloromethane (150 ml), washed with 2M HCl (20 ml), followed by aq. sodium bicarbonate (30 ml), dried (MgSO₄), and concentrated *in vacuo* to afford a syrup. Chromatography (ethyl acetate:petroleum ether, 1:2) gave pyrrole (**2f**), 0.508 g (68.6 %) as a clear syrup. ¹H NMR (CDCl₃) δ 7.86 (s, 1H), 7.13-7.31 (m, 15H), 6.35 (d, *J* = 3.1 Hz, 1H), 3.18 (t, 2H); 2.49 (t, 2H); ¹³C NMR δ 142.9 (C), 141.8 (C), 127.7 (CH), 126.8 (CH), 126.1 (CH), 121.3 (CH), 114.2 (C), 110.3 (C), 86.2 (C), 63.4 (CH₂), 23.9 (CH₂).

### 7-(2-(Trityloxy)ethyl)-5H-pyrrolo[3,2-d]pyrimidin-4-amine (2g)

Pyrrole (2f) (0.480 g, 1.220 mmol) was dissolved in abs EtOH (15 ml). Formamidine acetate (0.635 g, 6.10 mmol) was added and the reaction mixture was heated under reflux for 4 hrs. The solution was concentrated to dryness. Chromatography (ethyl acetate) gave (**2g**), 0.42 g (82 %) as a solidified syrup. ¹H NMR (MeOH-d₄) δ 8.47 (s, 1H), 7.54 (s, 1H), 7.12-7.41 (m, 15H), 3.30-3.35 (m, 2H); 3.0 (t, 2H); ¹³C NMR δ 152.8 (C), 149.6 (CH), 146.0 (C), 144.6 (C), 130.2 (CH), 130.0 (CH), 129.0 (CH), 128.3 (CH), 115.3 (C), 114.0 (C), 88.2 (C), 65.1 (CH₂), 25.8 (CH₂).

### N-Benzoyl-N-(7-(2-(trityloxy)ethyl)-5H-pyrrolo[3,2-d]pyrimidin-4-yl)benzamide (2h)

Pyrrolo-pyrimidine (2f) (0.4 g, 0.951 mmol) was dissolved in dry pyridine (15 ml) and cooled to 0 °C. Benzoyl chloride (2 ml, 17.22 mmol) was added and the reaction mixture was stirred at RT for 17 hrs. The resulting solution was diluted with dichloromethane, washed with water, followed by aq. sodium bicarbonate, dried (MgSO₄), and concentrated *in vacuo* to afford a syrup. Chromatography (ethyl acetate:petroleum ether, 1:4) gave over-*N*-benzoylated material as a syrup. This was dissolved in dry MeOH (20 ml) and treated with triethylamine (1 ml). The solution was stirred at RT for 17 hrs and concentrated to dryness. Chromatography (ethyl acetate:petroleum ether, 1:2) gave (**2h**), 0.53 g (89%) as a white foam. ¹H NMR (CDCl₃) d 8.4 (s, 1H), 8.1 (m, 1H), 7.8-8.0 (m, 4H), 7.13-7.49 (m, 21H), 3.4 (t, 2H); 3.1 (t, 2H); ¹³C NMR d 171.5 (C), 167.5 (C), 151.7 (C), 148.8 (CH), 144.8 (C), 142.8 (C), 133.8 (CH), 132.2 (CH), 131.3 (CH), 130.4 (CH), 130.2 (CH), 129.1 (CH), 128.7 (CH), 128.5 (CH), 128.1 (CH), 127.3 (CH), 116.3 (C), 114.4 (C), 87.0 (C), 63.9 (CH₂), 24.9 (CH₂).

### N-Benzoyl-N-(7-(2-hydroxyethyl)-5H-pyrrolo[3,2-d]pyrimidin-4-yl)benzamide (2i)

*N*-Benzoyl derivative (**2h**) (0.2 g, 0.318 mmol) was dissolved in aq. acetic acid (80%, 5 ml) and stirred at 60 °C for 4 hrs. The resulting solution was concentrated *in vacuo* to afford a syrup. Chromatography (ethyl acetate:pethroleum ether, 1:1) gave (**9**), 111 mg (90%) as a clear syrup.

### N-Benzoyl-N-(7-(2-oxoethyl)-5H-pyrrolo[3,2-d]pyrimidin-4-yl)benzamide (2j)

Alcohol (**2i**) (78 mg, 202 µmol) was dissolved in dry dichloromethane (5 ml) and treated with Dess-Martin periodinane (1.5 eq., 128 mg). The reaction mixture was stirred at RT for 1 hr. The resulting solution was diluted with ether and treated with 1M NaOH. After 15 min the organic layer was washed with water, dried (MgSO₄) and concentrated *in vacuo* to afford a syrup. Chromatography (ethyl acetate:petroleum ether, 1:1) gave (2j), 71 mg (92%) as a clear syrup.

### (3S,4R)-1-[2-(9-Deaza-adenin-9-yl)ethyl]-3-hydroxy-4-methylthiomethylpyrrolidine (2) [Methylthio-DAD-Et-Immucillin-A]

Acetaldehdo-derivative (**2j**) can be coupled with the (3S,4R)-3-hydroxy-4-methylthiomethylpyrrolidine (**2k**) by reductive amination using sodium cyanoborohydride in methanol at room temperature, following methodology reported in Evans et al, J. Med. Chem., 48 (2005) 4679 - 4689, (see Scheme 1), and the N-benzoyl protecting groups can then be removed by treatment of the product with methanolic ammonia, to yield the title compound (**2**).

### Example 3: Inhibition Studies

Initial (*K*ᵢ) and equilibrium (*K*_{¡}*) dissociation constants of DAD-Et-Immucillin-H were determined for human PNP.

Inhibitor dissociation constants for the phosphorolysis of inosine were based on initial and equilibrium reaction rate measurements with varied inhibitor concentrations (Miles, R. W., Tyler, P. C., Furneaux, R. H., Bagdassarian, C. K. and Schramm, V. L. (1998) One-third-the-sites transition state inhibitors for purine nucleoside phosphorylase, Biochemistry 37, 8615-8621; Morrison, J. F. and Walsh, C. T. (1988) The behaviour and significance of slow-binding enzyme inhibitors, Adv. Enzymol. Relat. Areas Mol. Biol. 61, 201-301). Reactions were started by adding huPNP (1.4 nM) to reaction mixtures (25 °C) containing 1 mM inosine in 50 mM KHPO₄ pH 7.4 with xanthine oxidase at 60 mU/ml. Hypoxanthine formed by phosphorolysis of inosine was oxidized to uric acid and monitored spectrophotometrically at 293 nm (extinction coefficient for uric acid ε₂₉₃=12.9 mM⁻¹ cm⁻¹). Enzyme concentration was adjusted to give absorbance changes not exceeding 1.0 during the time required to characterize initial and final slow-onset inhibition equilibria. The large excess of substrate and continuous product depletion provided extended initial rate conditions. In most cases the concentration of the inhibitor compound was >10-fold greater than the enzyme concentration as required for simple analysis of two-state slow-onset tight-binding inhibition (Morrison, J. F. and Walsh, C. T. (1988) The behavior and significance of slow-binding enzyme inhibitors, Adv. Enzymol. Relat. Areas Mol. Biol. 61, 201-301). The inhibition constant *K*ᵢ describes the reversible equilibrium between enzyme and inhibitor (compound 1) for the initial inhibitor binding step. *K*ᵢ was determined by fitting the initial rates at different inhibitor concentration to the equation for competitive inhibition: υᵢ = (*k*_{cat} x S)/(*K*ₘ(1 +I/*k*ᵢ) + S), where υᵢ is initial reaction rate, *k*_{cat} is the catalytic turnover number, *K*ₘ is the Michaelis constant, *K*ᵢ is the dissociation constant of enzyme-inhibitor complex (EI), I is inhibitor concentration and S is substrate concentration. The dissociation constant for the complex formed after slow onset equilibrium (*K*ᵢ*) was determined by υ = (*k*_{cat} x S)/(*K*ₘ(1+I/*K*ᵢ*) + S), where υ is the steady state reaction rate and the other variables are the same as above.

Initial (*K*ᵢ) and equilibrium (*K*ᵢ*) dissociation constants of Compound 1 for huPNP were found to be 1.6 ± 0.3 nM and 0.46 ± 0.05 nM, respectively.

Although the invention has been described by way of example, it should be appreciated the variations or modifications may be made without departing from the scope of the invention.

### INDUSTRIAL APPLICABILITY

The present invention relates to compounds that are inhibitors of PNP, PPRT, MTAP, MTAN and/or NH. The compounds are therefore expected to be useful in the treatment of diseases in which the inhibition of PNP, PPRT, MTAP, MTAN and/or NH is desirable. Such diseases include cancer, and bacterial infection, protozoal infection or T-cell mediated diseases.

## Claims

1. A compound of the formula (I): 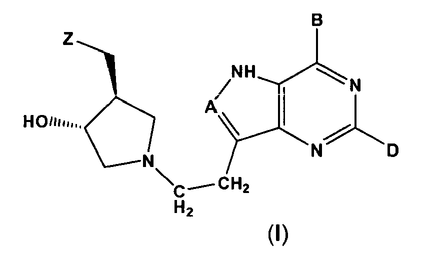 where:
A is N or CH;
B is OH or NH₂;
D is H, OH, NH₂ or SCH₃; and
Z is OH or SQ, where Q is an alkyl, aralkyl, or aryl group, each of which is optionally substituted with one or more substituents selected from an alkyl group, an alkoxy group, a halogen atom, an amino group, a carboxylic acid group, a carboxylate alkyl ester group and
an alkylthio group;
or a tautomer thereof; or a pharmaceutically acceptable salt thereof; or an ester form thereof.

2. A compound as claimed in claim 1 where A is CH.

3. A compound as claimed in claim 1 where A is N.

4. A compound as claimed in any one of claims 1 to 3 where B is OH.

5. A compound as claimed in any one of claims 1 to 3 where B is NH₂.

6. A compound as claimed in any one of claims 1 to 5 where D is H.

7. A compound as claimed in claim 1 where A is CH and D is H.

8. A compound as claimed in any one of claims 1 to 6 where D is NH₂, OH, or SCH₃.

9. A compound as claimed in any one of the preceding claims where Z is OH.

10. A compound as claimed in any one of claims 1 to 8 where Z is SQ.

11. A compound as claimed in claim 1 where Z is OH, A is CH, B is OH, and D is H or NH₂.

12. A compound as claimed in claim 1 where Z is SQ, A is CH, B is NH₂, and D is H.

13. A compound as claimed in claim 1 which is selected from the group:
(i) (3S,4S)-1-[(9-deazaadenin-9-yl)ethyl]-3-hydroxy-4-(hydroxymethyl)-pyrrolidine;
(ii) (3S,4R)-1-[(9-deazaadenin-9-yl)ethyl]-3-hydroxy-4-(methylthiomethyl)-pyrrolidine;
(iii) (3S,4R)-1-[(9-deazaadenin-9-yl)ethyl]-3-hydroxy-4-(ethylthiomethyl)-pyrrolidine;
(iv) (3S,4R)-1-[(9-deazaadenin-9-yl)ethyl]-3-hydroxy-4-(2-fluoroethylthiomethyl)-pyrrolidine;
(v) (3S,4R)-1-[(9-deazaadenin-9-yl)ethyl]-3-hydroxy-4-(2-hydroxyethy)thiomethyl)-pyrrolidine;
(vi) (3S,4R)-1-[(9-deazaadenin-9-yl)ethyl]-3-hydroxy-4-(propylthiomethyl)-pyrrolidine;
(vii) (3S,4R)-1-[(9-deazaadenin-9-yl)ethyl]-3-hydroxy-4-(isopropylthiomethyl)-pyrrolidine;
(viii) (3S,4R)-1-[(9-deazaadenin-9-yl)ethyl]-3-hydroxy-4-(butylthiomethyl)-pyrrolidine;
(ix) (3S,4R)-1-[(9-deazaadenin-9-yl)ethyl]-3-hydroxy-4-(cyclohexylylthiomethyl)-pyrrolidine;
(x) (3S,4R)-1-[(9-deazaadenin-9-yl)ethyl]-3-hydroxy-4-(cyclohexylmethylthiomethyl)-pyrrolidine;
(xi) (3S,4R)-1-[(9-deazaadenin-9-yl)ethyl]-3-hydroxy-4-(cyclopentylthiomethyl)-pyrrolidine;
(xii) (3S,4R)-1-[(9-deazaadenin-9-yl)ethyl]-3-hydroxy-4-(phenylthiomethyl)-pyrrolidine;
(xiii) (3S,4R)-1-[(9-deazaadenin-9-yl)ethyl]-3-hydroxy-4-(4-fluorophenylthiomethyl)-pyrrolidine;
(xiv) (3S,4R)-1-[(9-deazaadenin-9-yl)ethyl]-3-hydroxy-4-(4-chlorophenylthiomethyl)-pyrrolidine;
(xv) (3S,4R)-1-[(9-deazaadenin-9-yl)ethyl]-3-hydroxy-4-(3-chlorophenylthiomethyl)-pyrrolidine;
(xvi) (3S,4R)-1-[(9-deazaadenin-9-yl)ethyl]-3-hydroxy-4-(4-methylphenylthiomethyl)-pyrrolidine;
(xvii) (38,4 R)-1-[(9-deazaaden in-9-yl)ethyl]-3-hydroxy-4-(3-methylphenylthiomethyl)-pyrrolidine;
(xviii) (3S,4R)-1-[(9-deazaadenin-9-yl)ethyl]-3-hydroxy-4-(benzylthiomethyl)-pyrrolidine;
(xix) (3S,4S)-1-[(9-deazaguanin-9-yl)ethyl]-3-hydroxy-4-(hydroxymethyl)-pyrrolidine;
(xx) (3S,4R)-1-[(9-deazaguanin-9-yl)ethyl]-3-hydroxy-4-(methylthiomethyl)-pyrrolidine;
(xxi) (3S,4S)-1-[(9-deazahypoxanthin-9-yl)ethyl]-3-hydroxy-4-(hydroxymethyl)-pyrrolidine;
(xxii) (3S,4R)-1-[(9-deazahypoxanthin-9-yl)ethyl]-3-hydroxy-4-(methylthiomethyl)-pyrrolidine;
(xxiii) (3S,4S)-1-[(9-deazaxanthin-9-yl)ethyl]-3-hydroxy-4-(hydroxymethyl)-pyrrolidine;
(xxiv) (3S,4S)-1-[(9-deazaxanthin-9-yl)ethyl]-3-hydroxy-4-(methylthiomethyl)-pyrrolidine;
(xxv) (3S,4S)-1-[(8-aza-9-deazaadenin-9-yl)ethyl]-3-hydroxy-4-(hydroxymethyl)-pyrrolidine;
(xxvi) (35,45)-1-[(8-aza-9-deazaadenin-9-yl)ethyl]-3-hydroxy-4-methyl-pyrrolidine;
(xxvii) (3S,4R)-1-[(8-aza-9-deazaadenin-9-yl)ethyl]-3-hydroxy-4-(benzylthiomethyl)-pyrrolidine;
(xxviii) (3S,4R)-1-[(8-aza-9-deazaadenin-9-yl)ethyl]-3-hydroxy-4-(methylthiomethyl)-pyrrolidine;
(xxix) (3S,4R)-1-[(8-aza-9-deazaadenin-9-yl)ethyl]-3-hydroxy-4-(ethylthiomethyl)-pyrrolidine;
(xxx) (3S,4R)-1-[(8-aza-9-deazaadenin-9-yl)ethyl]-3-hydroxy-4-(propylthiomethyl)-pyrrolidine;
(xxxi) (3S,4R)-1-[(8-aza-9-deazaadenin-9-yl)ethyl]-3-hydroxy-4-(isopropylthiomethyl)-pyrrolidine;
(xxxii) (3S,4R)-1-[(8-aza-9-deazaadenin-9-yl)ethyl]-3-hydroxy-4-(butylthiomethyl)-pyrrolidine;
(xxxiii) (3S,4R)-1-[(8-aza-9-deazaadenin-9-yl)ethyl]-3-hydroxy-4-(phenylthiomethyl)-pyrrolidine;
(xxxiv) (3S,4R)-1-[(8-aza-9-deazaadenin-9-yl)ethyl]-3-hydroxy-4-(4-fluorophenylthiomethyl)-pyrrolidine;
(xxxv) (3S,4R)-1-[(8-aza-9-deazaadenin-9-yl)ethyl]-3-hydroxy-4-(4-chlorophenylthiomethyl)-pyrrolidine;
(xxxvi) (3S,4R)-1-[(8-aza-9-deazaadenin-9-yl)ethyl]-3-hydroxy-4-(3-chlorophenylthiomethyl)-pyrrolidine;
(xxxvii) (3S,4R)-1-[(8-aza-9-deazaadenin-9-yl)ethyl]-3-hydroxy-4-(4-methylphenylthiomethyl)-pyrrolidine;
(xxxviii)(3S,4R)-1-[(8-aza-9-deazaadenin-9-yl)ethyl]-3-hydroxy-4-(3-methylphenylthiomethyl)-pyrrolidine;
(xxxix) (3S,4S)-1-[(8-aza-9-deazaguanin-9-yl)ethyl]-3-hydroxy-4-(hydroxymethyl)-pyrrolidine;
(xl) (3S,4S)-1-[(8-aza-9-deazaguanin-9-yl)ethyl]-3-hydroxy-4-methyl-pyrrolidine;
(xli) (3S,4S)-1-[(8-aza-9-deazaguanin-9-yl)ethyl]-3-hydroxy-4-(methylthiomethyl)-pyrrolidine;
(xlii) (3S,4S)-1-[(8-aza-9-deazahypoxanthin-9-yl)ethyl]-3-hydroxy-4-(hydroxymethyl)-pyrrolidine;
(xliii) (3S,4S)-1-[(8-aza-9-deazahypoxanthin-9-yl)ethyl]-3-hydroxy-4-methyl-pyrrolidine;
(xliv) (3S,4S)-1-[(8-aza-9-deazahypoxanthin-9-yl)ethyl]-3-hydroxy-4-(methylthiomethyl)-pyrrolidine;
(xlv) (3S,4S)-1-[(8-aza-9-deazaxanthin-9-yl)ethyl]-3-hydroxy-4-(hydroxymethyl)-pyrrolidine; and
(xlvi) (3S,4S)-1-[(8-aza-9-deazaxanthin-9-yl)ethyl]-3-hydroxy-4-(methylthiomethyl)-pyrrolidine.

14. A pharmaceutical composition comprising a pharmaceutically effective amount of a compound of claim 1.

15. A pharmaceutical composition as claimed in claim 14 where the compound of claim 1 is one where Z is OH, A is CH, B is OH, and D is H or NH₂.

16. A pharmaceutical composition as claimed in claim 14 where the compound of claim 1 is one where Z is SQ, A is CH, B is NH₂, and D is H.

17. A compound of any one of claims 1 to 13 for use as a medicament.

18. A compound of any one of claims 1 to 13 for use in the treatment of a disease or condition in which it is desirable to inhibit purine phosphoribosyltransferase, purine nucleoside phosphorylase, 5'-methylthioadenosine phosphorylase, 5'-methylthioadenosine nucleosidase and/or nucleoside hydrolase.

19. The use of a compound as claimed in any one of claims 1 to 13 in the manufacture of a medicament for treating a disease or condition in which it is desirable to inhibit purine phosphoribosyltransferase, purine nucleoside phosphorylase, 5'-methylthioadenosine phosphorylase, 5'-methylthioadenosine nucleosidase and/or nucleoside hydrolase.

20. The compound for use as claimed in claim 18 or the use as claimed in claim 19 where the disease or condition is cancer, bacterial infection, protozoal infection or a T-cell mediated disease.

21. The compound for use or use as claimed in claim 20 where the T-cell mediated disease is psoriasis, arthritis or transplant rejection.

22. The compound for use or use as claimed in any one of claims 17 to 21 where the compound of claim 1 is one where Z is OH, A is CH, B is OH, and D is H or NH₂.

23. The compound for use or use as claimed in any one of claims 17 to 21 where the compound of claim 1 is one where Z is SQ, A is CH, B is NH₂, and D is H.

24. A method of preparing a compound according to claim 1 where a 2-(9-deaza-puine-9-yl)acetaldehyde, or protected form thereof, is coupled by reductive amination to (3R,4S)-3-hydroxy-4-hydroxymethylpyrrolidine.

25. A method of preparing a compound according to claim 1 where a 2-(9-deaza-purine-9-yl)acetaldehyde, or protected form thereof, is coupled by reductive amination to a (3R,4S)-3-hydroxy-4-alkyl-, 4-aralkyl- or arylthiomethylpyrrolidine, where the alkyl-, aralkyl- or aryl groups are each optionally substituted.

## Patentansprüche

1. Verbindung der Formel (I) 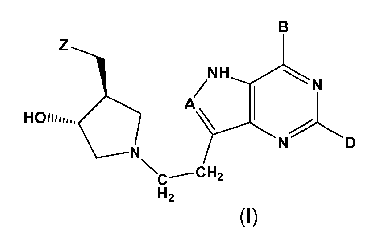 wobei:
A N oder CH ist;
B OH oder NH₂ ist;
D H, OH, NH₂ oder SCH₃ ist; und
Z OH oder SQ ist, wobei Q ein Alkyl, Aralkyl, oder eine Arylgruppe ist, jede davon gegebenenfalls substituiert mit einer oder mehreren Substituenten ausgewählt aus der Gruppe bestehend aus einer Alkylgruppe, einer Alkoxygruppe, einem Halogenatom, einer Aminogruppe, einer Carbonsäuregruppe, einer Carboxylatalkylestergruppe und einer Alkylthiogruppe;
oder einem Tautomeren davon; oder ein pharmazeutisch geeignetes Salz davon; oder einem Ester davon.

2. Verbindung gemäß Anspruch 1, wobei A CH ist.

3. Verbindung gemäß Anspruch 1, wobei A N ist.

4. Verbindung gemäß einem der Ansprüche 1 bis 3, wobei B OH ist.

5. Verbindung gemäß einem der Ansprüche 1 bis 3, wobei B NH₂ ist.

6. Verbindung gemäß einem der Ansprüche 1 bis 5, wobei D H ist.

7. Verbindung gemäß Anspruch 1, wobei A CH ist und D H ist.

8. Verbindung gemäß einem der Ansprüche 1 bis 6, wobei D NH₂, OH oder SCH₃ ist.

9. Verbindung gemäß einem der vorherigen Ansprüche, wobei Z OH ist.

10. Verbindung gemäß einem der Ansprüche 1 bis 8, wobei Z SQ ist.

11. Verbindung gemäß Anspruch 1 wobei Z OH ist, A CH ist, B OH ist, und D H oder NH₂ ist.

12. Verbindung gemäß Anspruch 1, wobei Z SQ ist, A CH ist, B NH₂ ist, und D H ist.

13. Verbindung gemäß Anspruch 1, ausgewählt aus der Gruppe bestehend aus:
(i) (3S,4S)-1-[(9-Deazaadenin-9-yl)ethyl]-3-hydroxy-4-(hydroxymethyl)-pyrrolidin;
(ii) (3S,4R)-1-[(9-Deazaadenin-9-yl)ethyl]-3-hydroxy-4-(methylthiomethyl)-pyrrolidin;
(iii) (3S,4R)-1-[(9-Deazaadenin-9-yl)ethyl]-3-hydroxy-4-(ethylthiomethyl)-pyrrolidin;
(iv) (3S,4R)-1-[(9-Deazaadenin-9-yl)ethyl]-3-hydroxy-4-(2-fluoroethylthiomethyl)-pyrrolidin;
(v) (3S,4S)-1-[(9-Deazaadenin-9-yl)ethyl]-3-hydroxy-4-(2-hydroxyethylthiomethyl)-pyrrolidin;
(vi) (3S,4R)-1-[(9-Deazaadenin-9-yl)ethyl]-3-hydroxy-4-(propylthiomethyl)-pyrrolidin;
(vii) (3S,4R)-1-[(9-Deazaadenin-9-yl)ethyl]-3-hydroxy-4-(isopropylthiomethyl)-pyrrolidin;
(viii) (3S,4R)-1-[(9-Deazaadenin-9-yl)ethyl]-3-hydroxy-4-(butylthiomethyl)-pyrrolidin;
(ix) (3S,4R)-1-[(9-Deazaadenin-9-yl)ethyl]-3-hydroxy-4-(cyclohexylthiomethyl)-pyrrolidin;
(x) (3S,4R)-1-[(9-Deazaadenin-9-yl)ethyl]-3-hydroxy-4-(cyclohexylmethylthiomethyl)-pyrrolidin;
(xi) (3S,4R)-1-[(9-Deazaadenin-9-yl)ethyl]-3-hydroxy-4-(cyclopentylthiomethyl)-pyrrolidin;
(xii) (3S,4R)-1-[(9-Deazaadenin-9-yl)ethyl]-3-hydroxy-4-(phenylthiomethyl)-pyrrolidin;
(xiii) (3S,4R)-1-[(9-Deazaadenin-9-yl)ethyl]-3-hydroxy-4-(4-fluorophenylthiomethyl)-pyrrolidin;
(xiv) (3S,4R)-1-[(9-Deazaadenin-9-yl)ethyl]-3-hydroxy-4-(4-chlorophenylthiomethyl)-pyrrolidin;
(xv) (3S,4R)-1-[(9-Deazaadenin-9-yl)ethyl]-3-hydroxy-4-(3-chlorophenylthiomethyl)-pyrrolidin;
(xvi) (3S,4R)-1-[(9-Deazaadenin-9-yl)ethyl]-3-hydroxy-4-(4-methylphenylthiomethyl)-pyrrolidin;
(xvii) (3S,4R)-1-[(9-Deazaadenin-9-yl)ethyl]-3-hydroxy-4-(3-methylphenylthiomethyl)-pyrrolidin;
(xviii) (3S,4R)-1-[(9-Deazaadenin-9-yl)ethyl]-3-hydroxy-4-(benzylthiomethyl)-pyrrolidin;
(xix) (3S,4S)-1-[(9-Deazaguanin-9-yl)ethyl]-3-hydroxy-4-(hydroxymethyl)-pyrrolidin;
(xx) (3S,4R)-1-[(9-Deazaguanin-9-yl)ethyl]-3-hydroxy-4-(methylthiomethyl)-pyrrolidin;
(xxi) (3S,4S)-1-[(9-Deazahypoxanthin-9-yl)ethyl]-3-hydroxy-4-(hydroxymethyl)-pyrrolidin;
(xxii) (3S,4R)-1-[(9-Deazahypoxanthin-9-yl)ethyl]-3-hydroxy-4-(methylthiomethyl)-pyrrolidin;
(xxiii) (3S,4S)-1-[(9-Deazaxanthin-9-yl)ethyl]-3-hydroxy-4-(hydroxymethyl)-pyrrolidin;
(xxiv) (3S,4S)-1-[(9-Deazaxanthin-9-yl)ethyl]-3-hydroxy-4-(methylthiomethyl)-pyrrolidin;
(xxv) (3S,4S)-1-[(8-Aza-deazaadenin-9-yl)ethyl]-3-hydroxy-4-(hydroxymethyl)-pyrrolidin;
(xxvi) (3S,4S)-1-[(8-Aza-9-deazaadenin-9-yl)ethyl]-3-hydroxy-4-methyl-pyrrolidin;
(xxvii) (3S,4R)-1-[(8-Aza-9-deazaadenin-9-yl)ethyl]-3-hydroxy-4-(benzylthiomethyl)-pyrrolidin;
(xxviii)(3S,4R)-1-[(8-Aza-9-deazaadenin-9-yl)ethyl]-3-hydroxy-4-(methylthiomethyl)-pyrrolidin;
(xxix) (3S,4R)-1-[(8-Aza-9-deazaadenin-9-yl)ethyl]-3-hydroxy-4-(ethylthiomethyl)-pyrrolidin;
(xxx) (3S,4R)-1-[(8-Aza-9-deazaadenin-9-yl)ethyl]-3-hydroxy-4-(propylthiomethyl)-pyrrolidin;
(xxxi) (3S,4R)-1-[(8-Aza-9-deazaadenin-9-yl)ethyl]-3-hydroxy-4-(isopropylthiomethyl)-pyrrolidin;
(xxxii) (3S,4R)-1-[(8-Aza-9-deazaadenin-9-yl)ethyl]-3-hydroxy-4-(butylthiomethyl)-pyrrolidin;
(xxxiii)(35,4R)-1-[(8-Aza-9-deazaadenin-9-yl)ethyl]-3-hydroxy-4-(phenylthiomethyl)-pyrrolidin;
(xxxiv)(3S,4R)-1-[(8-Aza-9-deazaadenin-9-yl)ethyl]-3-hydroxy-4-(4-fluorophenylthio-methyl)-pyrrolidin;
(xxxv) (3S,4R)-1-[(8-Aza-9-deazaadenin-9-yl)ethyl]-3-hydroxy-4-(4-chlorophenylthio-methyl)-pyrrolidin;
(xxxvi)(3S,4R)-12-[(8-Aza-9-deazaadenin-9-yl)ethyl]-3-hydroxy-4-(3-chlorophenylthio-methyl)-pyrrolidin;
(xxxvii) (3S,4R)-1-[(8-Aza-9-deazaadenin-9-yl)ethyl]-3-hydroxy-4-(4-methyl-phenylthiomethyl)-pyrrolidin;
(xxxviii)(3S,4R)-1-[(8-Aza-9-deazaadenin-9-yl)ethyl]-3-hydroxy-4-(3-methyl-phenylthiomethyl)-pyrrolidin;
(xxxix)(3S,4S)-l-[(8-Aza-9-deazaguanin-9-yl)ethyl]-3-hydroxy-4-(hydroxymethyl)-pyrrolidin;
(xl) (3S,4S)-1-[(8-Aza-9-deazaguanin-9-yl)ethyl]-3-hydroxy-4-methyl-pyrrolidin;
(xli) (3S,4S)-1-[(8-Aza-9-deazaguanin-9-yl)ethyl]-3-hydroxy-4-(methylthiomethyl)-pyrrolidin;
(xlii) (3S,4S)-1-[(8-Aza-9-deazahypoxanthin-9-yl)ethyl]-3-hydroxy-4-(hydroxymethyl)-pyrrolidin;
(xliii) (3S,4S)-1-[(8-Aza-9-deazahypoxanthin-9-yl)ethyl]-3-hydroxy-4-methylpyrrolidin;
(xliv) (3S,4S)-1-[(8-Aza-9-deazahypoxanthin-9-yl)ethyl]-3-hydroxy-4-(methylthiomethyl)-pyrrolidin;
(xlv) (3S,4S)-1-[(8-Aza-9-deazaxanthin-9-yl)ethyl]-3-hydroxy-4-(hydroxymethyl)-pyrrolidin; und
(xlvi) (3S,4S)-1-[(8-Aza-9-deazaxanthin-9-yl)ethyl]-3-hydroxy-4-(methylthiomethyl)-pyrrolidin;

14. Pharmazeutische Zusammensetzung umfassend eine pharmazeutische wirksame Menge einer Verbindung gemäß Anspruch 1.

15. Pharmazeutische Zusammensetzung gemäß Anspruch 14, wobei die Verbindung gemäß Anspruch 1 eine Verbindung ist bei der Z OH ist, A CH ist, B OH ist, und D H oder NH₂ ist.

16. Pharmazeutische Zusammensetzung gemäß Anspruch 14, wobei die Verbindung gemäß Anspruch 1 eine Verbindung ist bei der Z SQ ist, A CH ist, B NH₂ ist, und D H ist.

17. Verbindung gemäß einem der Ansprüche 1 bis 13, zur Verwendung als Medikament.

18. Verbindung gemäß einem der Ansprüche 1 bis 13 zur Verwendung bei der Behandlung einer Krankheit oder eines Zustandes bei der/dem wünschenswert ist Purinphosphoribosyltransferase, Purinnukleosidphosphorylase, 5'-Methylthioadenosinphosphorylase, 5'-Methylthioadenosinnukleosidase und/oder Nukleosidhydrolase zu hemmen.

19. Verwendung einer Verbindung gemäß einem der Ansprüche 1 bis 13 zur Herstellung eines Medikaments zur Behandlung einer Krankheit oder eines Zustandes bei der/dem wünschenswert ist Purinphosphoribosyltransferase, Purinnukleosidphosphorylase, 5'-Methylthioadenosinphosphorylase, 5'-Methylthioadenosinnukleosidase und/oder Nukleosidhydrolase zu hemmen.

20. Verbindung zur Verwendung gemäß Anspruch 18 oder zur Verwendung gemäß Anspruch 19, wobei die Krankheit oder der Zustand Krebs, eine bakterielle Infektion, Protozoeninfektion oder eine T-Zell-vermittelte Erkrankung ist.

21. Verbindung zur Verwendung oder Verwendung gemäß Anspruch 20, wobei die T-zellenvermittelte Krankheit Psoriasis, Arthritis oder Transplantatabstoßung ist.

22. Verbindung zur Verwendung oder Verwendung gemäß einem der Ansprüche 17 bis 21, wobei die Verbindung gemäß Anspruch 1 eine Verbindung ist bei der Z OH ist, A CH ist, B OH ist, und D H oder NH₂ ist.

23. Verbindung zur Verwendung oder Verwendung gemäß einem der Ansprüche 17 bis 21, wobei die Verbindung gemäß Anspruch 1 eine Verbindung ist bei der Z SQ ist, A CH ist, B NH₂ ist, und D H ist.

24. Methode zur Herstellung einer Verbindung gemäß Anspruch 1, wobei ein 2-(9-Deazapurin-9-yl)acetaldehyd, oder eine geschützte Form davon, durch reduktive Aminierung an (3R,4S)-3-Hydroxy-4-hydroxymethylpyrrolidin gekuppelt wird.

25. Methode zur Herstellung einer Verbindung gemäß Anspruch 1, wobei ein 2-(9-Deazapurin-9-yl)acetaldehyd, oder eine geschützte Form davon, durch reduktive Aminierung an ein (3R,4S)-3-Hydroxy-4-alkyl-, 4-Aralkyl- oder Arylthiomethylpyrrolidin, wobei jede Alkyl-, Aralkyl- oder Arylgruppe gegebenenfalls substituiert ist, gekuppelt wird.

## Revendications

1. Composé de formule (I) : 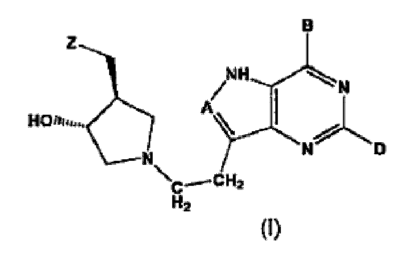 où :
A est N ou CH ;
B est OH ou NH₂ ;
D est H, OH, NH₂ ou SCH₃ ; et
Z est OH ou SQ, où Q est un groupe alkyle, aralkyle ou aryle, chacun étant facultativement substitué avec un ou plusieurs substituants choisis parmi un groupe alkyle, un groupe alcoxy, un atome d'halogène, un groupe amino, un groupe acide carboxylique, un groupe ester carboxylate d'alkyle et un groupe alkylthio ;
ou un de ses tautomères ; ou un de ses sels pharmaceutiquement acceptables ; ou une de ses formes d'ester.

2. Composé selon la revendication 1, dans lequel A est CH.

3. Composé selon la revendication 1, dans lequel A est N.

4. Composé selon l'une quelconque des revendications 1 à 3, dans lequel B est OH.

5. Composé selon l'une quelconque des revendications 1 à 3, dans lequel B est NH₂.

6. Composé selon l'une quelconque des revendications 1 à 5, dans lequel D est H.

7. Composé selon la revendication 1, dans lequel A est CH et D est H.

8. Composé selon l'une quelconque des revendications 1 à 6, dans lequel D est NH₂, OH ou SCH₃.

9. Composé selon l'une quelconque des revendications précédentes, dans lequel Z est OH.

10. Composé selon l'une quelconque des revendications 1 à 8, dans lequel Z est SQ.

11. Composé selon la revendication 1, dans lequel Z est OH, A est CH, B est OH et D est H ou NH₂.

12. Composé selon la revendication 1, dans lequel Z est SQ, A est CH, B est NH₂ et D est H.

13. Composé selon la revendication 1, qui est choisi dans le groupe comprenant :
(i) la (3S,4S)-1-[(9-déazaadénin-9-yl)éthyl]-3-hydroxy-4-(hydroxyméthyl)-pyrrolidine ;
(ii) la (3S,4R)-1-[(9-déazaadénin-9-yl)éthyl]-3-hydroxy-4-(méthylthiométhyl)-pyrrolidine ;
(iii) la (3S,4R)-1-[(9-déazaadénin-9-yl)éthyl]-3-hydroxy-4-(éthylthiométhyl)-pyrrolidine ;
(iv) la (3S,4R)-1-[(9-déazaadénin-9-yl)éthyI]-3-hydroxy-4-(2-fluoroéthylthiométhyl)-pyrrolidine ;
(v) la (3S,4R)-1-[(9-déazaadénin-9-yl)éthyI]-3-hydroxy-4-(2-hydroxyéthylthiométhyl)-pyrrolidine ;
(vi) la (3S,4R)-1-[(9-déazaadénin-9-yl)éthyl]-3-hydroxy-4-(propylthiométhyl)-pyrrolidine ;
(vii) la (3S,4R)-1-[(9-déazaadénin-9-yl)éthyl]-3-hydroxy-4-(isopropylthiométhyl)-pyrrolidine ;
(viii) la (3S,4R)-1-[(9-déazaadénin-9-yl)éthyl]-3-hydroxy-4-(butylthiométhyl)-pyrrolidine ;
(ix) la (3S,4R)-1-[(9-déazaadénin-9-yl)éthyl]-3-hydroxy-4-(cyclohexylylthiométhyl)-pyrrolidine ;
(x) la (3S,4R)-1-[(9-déazaadénin-9-yl)éthyl]-3-hydroxy-4-(cyclohexylméthylthiométhyl)-pyrrolidine ;
(xi) la (3S,4R)-1-[(9-déazaadénin-9-yl)éthyl]-3-hydroxy-4-(cyclopentylthiométhyl)-pyrrolidine ;
(xii) la (3S,4R)-1-[(9-déazaadénin-9-yl)éthyl]-3-hydroxy-4-(phénylthiométhyl)-pyrrolidine ;
(xiii) la (3S,4R)-1-[(9-déazaadénin-9-yl)éthyl]-3-hydroxy-4-(4-fluorophénylthiométhyl)-pyrrolidine ;
(xiv) la (3S,4R)-1-[(9-déazaadénin-9-yl)éthyl]-3-hydroxy-4-(4-chlorophénylthiométhyl)-pyrrolidine ;
(xv) la (3S,4R)-1-[(9-déazaadénin-9-yl)éthyl]-3-hydroxy-4-(3-chlorophénylthiométhyl)-pyrrolidine ;
(xvi) la (3S,4R)-1-[(9-déazaadénin-9-yl)éthyl]-3-hydroxy-4-(4-méthylphénylthiométhyl)-pyrrolidine ;
(xvii) la (3S,4R)-1-[(9-déazaadénin-9-yl)éthyl]-3-hydroxy-4-(3-méthylphénylthiométhyl)-pyrrolidine ;
(xviii) la (3S,4R)-1-[(9-déazaadénin-9-yl)éthyl]-3-hydroxy-4-(benzylthiométhyI)-pyrrolidine ;
(xix) la (3S,4S)-1-[(9-déazaguanin-9-yl)éthyl]-3-hydroxy-4-(hydroxyméthyl)-pyrrolidine ;
(xx) la (3S,4R)-1-[(9-déazaguanin-9-yl)éthyl]-3-hydroxy-4-(méthylthiométhyI)-pyrrolidine ;
(xxi) la (3S,4S)-1-[(9-déazahypoxanthin-9-yl)éthyl]-3-hydroxy-4-(hydroxyméthyl)-pyrrolidine ;
(xxii) la (3S,4R)-1-[(9-déazahypoxanthin-9-yl)éthyl]-3-hydroxy-4-(méthylthiométhyl)-pyrrolidine ;
(xxiii) la (3S,4S)-1-[(9-déazaxanthin-9-yl)éthyl]-3-hydroxy-4-(hydroxyméthyl)-pyrrolidine ;
(xxiv) la (3S,4S)-1-[(9-déazaxanthin-9-yl)éthyI]-3-hydroxy-4-(méthylthiométhyl)-pyrrolidine ;
(xxv) la (3S,4S)-1-[(8-aza-9-déazaadérün-9-yl)éthyI]-3-hydroxy-4-(hydroxyméthyl)-pyrrolidine ;
(xxvi) la (3S,4S)-1-[(8-aza-9-déazaadénin-9-yl)éthyl]-3-hydroxy-4-méthyl-pyrrolidine ;
(xxvii) la (3S,4R)-1-[(8-aza-9-déazaadénin-9-yl)éthyl]-3-hydroxy-4-(benzylthiométhyl)-pyrrolidine ;
(xxviii) la (3S,4R)-1-[(8-aza-9-déazaadénin-9-yl)éthyl]-3-hydroxy-4-(méthylthiométhyl)-pyrrolidine ;
(xxix) la (3S,4R)-1-[(8-aza-9-déazaadénin-9-yl)éthyl]-3-hydroxy-4-(éthylthiométhyl)-pyrrolidine ;
(xxx) la (3S,4R)-1-[(8-aza-9-déazaadénin-9-yl)éthyl]-3-hydroxy-4-(propylthiométhyl)-pyrrolidine ;
(xxxi) la (3S,4R)-1-[(8-aza-9-déazaadérün-9-yl)éthyl]-3-hydroxy-4-(isopropylthiométhyl)-pyrrolidine ;
(xxxii) la (3S,4R)-1-[(8-aza-9-déazaadénin-9-yl)éthyl]-3-hydroxy-4-(butylthio-méthyl)-pyrrolidine ;
(xxxiii) la (3S,4R)-1-[(8-aza-9-déazaadénin-9-yl)éthyl]-3-hydroxy-4-(phénylthio-méthyl)-pyrrolidine ;
(xxxiv) la (3S,4R)-1-[(8-aza-9-déazaadénin-9-yl)éthyl]-3-hydroxy-4-(4-fluoro-phénylthiométhyl)-pyrrolidine ;
(xxxv) la (3S,4R)-1-[(8-aza-9-déazaadérün-9-yl)éthyl]-3-hydroxy-4-(4-chlorophényl-thiométhyl)-pyrrolidine ;
(xxxvi) la (3S,4R)-1-[(8-aza-9-déazaadérün-9-yl)éthyl]-3-hydroxy-4-(3-chloro-phénylthiométhyl)-pyrrolidine ;
(xxxvii) la (3S,4R)-1-[(8-aza-9-déazaadérün-9-yl)éthyl]-3-hydroxy-4-(4-méthyl-phénylthiométhyl)-pyrrolidine ;
(xxxviii) la (3S,4R)-1-[(8-aza-9-déazaadénin-9-yl)éthyl]-3-hydroxy-4-(3-méthyl-phénylthiométhyl)-pyrrolidine ;
(xxxix) la (3S,4S)-1-[(8-aza-9-déazaguanin-9-yl)éthyl]-3-hydroxy-4-(hydroxy-méthyl)-pyrrolidine ;
(xl) la (3S,4S)-1-[(8-aza-9-déazaguanin-9-yl)éthyl]-3-hydroxy-4-méthyl-pyrrolidine ;
(xli) la (3S,4S)-1-[(8-aza-9-déazaguanin-9-yl)éthyl]-3-hydroxy-4-(méthylthio-méthyl)-pyrrolidine ;
(xlii) la (3S,4S)-1-[(8-aza-9-déazahypoxanthin-9-yl)éthyl]-3-hydroxy-4-(hydroxy-méthyl)-pyrrolidine ;
(xliii) la (3S,4S)-1-[(8-aza-9-déazahypoxanthin-9-yl)éthyl]-3-hydroxy-4-méthyl-pyrrolidine ;
(xliv) la (3S,4S)-1-[(8-aza-9-déazahypoxanthin-9-yl)éthyl]-3-hydroxy-4-(méthyl-thiométhyl)-pyrrolidine ;
(xlv) la (3S,4S)-1-[(8-aza-9-déazaxanthin-9-yl)éthyl]-3-hydroxy-4-(hydroxyméthyl)-pyrrolidine ; et
(xlvi) la (3S,4S)-1-[(8-aza-9-déazaxanthin-9-yl)éthyl]-3-hydroxy-4-(méthylthio-méthyl)-pyrrolidine.

14. Composition pharmaceutique comprenant une quantité pharmaceutiquement efficace d'un composé de la revendication 1.

15. Composition pharmaceutique selon la revendication 14, dans laquelle le composé de la revendication 1 est un composé dans lequel Z est OH, A est CH, B est OH et D est H ou NH₂.

16. Composition pharmaceutique selon la revendication 14, dans laquelle le composé de la revendication 1 est un composé dans lequel Z est SQ, A est CH, B est NH₂ et D est H.

17. Composé selon l'une quelconque des revendications 1 à 13, pour un usage comme médicament.

18. Composé selon l'une quelconque des revendications 1 à 13, pour un usage dans le traitement d'une maladie ou d'une affection dans laquelle il est souhaitable d'inhiber une purine phosphoribosyltransférase, une purine nucléoside phosphorylase, une 5'-méthylthioadénosine phosphorylase, une 5'-méthylthioadénosine nucléosidase et/ou un nucléoside hydrolase.

19. Utilisation d'un composé selon l'une quelconque des revendications 1 à 13, dans la préparation d'un médicament destiné au traitement d'une maladie ou d'une affection dans laquelle il est souhaitable d'inhiber une purine phosphoribosyltransférase, une purine nucléoside phosphorylase, une 5'-méthylthioadénosine phosphorylase, une 5'-méthylthioadénosine nucléosidase et/ou un nucléoside hydrolase.

20. Composé pour un usage selon la revendication 18 ou l'utilisation selon la revendication 19, la maladie ou l'affection étant un cancer, une infection bactérienne, une infection par un protozoaire ou une maladie induite par les lymphocytes T.

21. Composé pour un usage ou utilisation selon la revendication 20, dans lequel la maladie induite par les lymphocytes T est un psoriasis, une arthrite ou un rejet de greffe.

22. Composé pour un usage ou utilisation selon l'une quelconque des revendications 17 à 21, dans lequel le composé de la revendication 1 est un composé dans lequel Z est OH, A est CH, B est OH et D est H ou NH₂.

23. Composé pour un usage ou utilisation selon l'une quelconque des revendications 17 à 21, dans lequel le composé de la revendication 1 est un composé dans lequel Z est SQ, A est CH, B est NH₂ et D est H.

24. Procédé de préparation d'un composé selon la revendication 1, dans lequel un 2-(9-déaza-puine-9-yl)acétaldéhyde, ou une forme protégée de celui-ci, est couplé par amination réductrice à la (3R,4S)-3-hydroxy-4-hydroxyméthylpyrrolidine.

25. Procédé de préparation d'un composé selon la revendication 1, dans lequel un 2-(9-déazapurine-9-yl)acétaldéhyde, ou une forme protégé de celui-ci, est couplé par amination réductrice à une (3R,4S)-3-hydroxy-4-alkyl-, 4-aralkyl- ou arylthiométhylpyrrolidine, les groupes alkyle, aralkyle ou aryle étant chacun facultativement substitués.
